# EUROPEAN PATENT APPLICATION

(11) **EP 1 170 383 A2**
(43) Date of publication of application: **09.01.2002**
(21) Application number: 01113194.3
(22) Date of filing: 30.05.2001
(51) Int. Cl.: C12Q 1/70

(54) **Oligonucleotides and method for detection of small round structured virus (SRSV) RNA**

(30) Priority: 31.05.2000 JP 2000166502; 13.06.2000 JP 2000182326
(71) Applicant: Tosoh Corporation, Shinnanyo-shi, Yamaguchi-ken, 746-8501 (JP)
(72) Inventor: Saito, Juichi, Yamato-shi, Kanagawa-ken (JP); Masuda, Noriyoshi, Tokyo (JP); Ishiguro, Takahiko, Yokohama-shi, Kanagawa-ken (JP)
(74) Representative: Leson, Thomas Johannes Alois, Dipl.-Ing.

(57) **Abstract**

Oligonucleotides, for detection of small round structured virus (SRSV) RNA, which bind specifically to SRSV RNA at a relatively low, constant temperature (for example, 41°C), and an SRSV RNA detection method, which is a constant temperature nucleic acid amplification method employing the oligonucleotides, are provided.

SEQUENCE LISTING
<110> Tosoh Corporation

<120> Oligonucleotides and Method for Detection of Small
Round Structured Virus (SRSV) RNA

<130> 200-2509

<160> 38

<210> 1
<211> 20
<212> DNA
<213> Artificial Sequence

<220>
<223> oligonucleotide capable of binding specifically to SRSV
RNA

<400> 1
tccttttatg gtatgggtgt                                    20

<210> 2
<211> 20
<212> DNA
<213> Artificial Sequence

<220>
<223> oligonucleotide capable of binding specifically to SRSV
RNA

<400> 2
gggccgcggt gtaaagtggc                                    20

<210> 3
<211> 20
<212> DNA
<213> Artificial Sequence

<220>
<223> oligonucleotide capable of binding specifically to SRSV
RNA

<400> 3
cttctatgga gttcatacca                                                    20

<210> 4
<211> 20
<212> DNA
<213> Artificial Sequence

<220>
<223> oligonucleotide capable of binding specifically to SRSV
RNA

<400> 4
gctgagtaat ctgcatcaaa                                                    20

<210> 5
<211> 20
<212> DNA
<213> Artificial Sequence

<220>
<223> oligonucleotide capable of binding specifically to SRSV
RNA

<400> 5
ctaccattct ggaattgaac                                                    20

<210> 6
<211> 20
<212> DNA
<213> Artificial Sequence

<220>

<223> oligonucleotide capable of binding specifically to SRSV
RNA

<400> 6
attgggtttg acagaattgg                                                20

<210> 7
<211> 20
<212> DNA
<213> Artificial Sequence

<220>
<223> oligonucleotide capable of binding specifically to SRSV
RNA

<400> 7
tatagatgcc ataaaataca                                                20

<210> 8
<211> 23
<212> DNA
<213> Artificial Sequence

<220>
<223> oligonucleotide capable of binding specifically to SRSV
RNA

<400> 8
aaaggaaaaa tgatgattgg aat                                            23

<210> 9
<211> 23
<212> DNA
<213> Artificial Sequence

<220>
<223> oligonucleotide capable of binding specifically to SRSV
RNA

<400> 9
ataaaaggaa aaatgatgat tgg                                    23

<210> 10
<211> 23
<212> DNA
<213> Artificial Sequence

<220>
<223> oligonucleotide capable of binding specifically to SRSV
RNA

<400> 10
cggccctaaa ggatgaatta gtc                                    23

<210> 11
<211> 23
<212> DNA
<213> Artificial Sequence

<220>
<223> oligonucleotide capable of binding specifically to SRSV
RNA

<400> 11
ccgcggccct aaaggatgaa tta                                    23

<210> 12
<211> 23
<212> DNA
<213> Artificial Sequence

<220>
<223> oligonucleotide capable of binding specifically to SRSV
RNA

<400> 12

tggtaggtgt acaattgacg gtc 23

<210> 13
<211> 23
<212> DNA
<213> Artificial Sequence

<220>
<223> oligonucleotide capable of binding specifically to SRSV
RNA

<400> 13
gaatggtagg tgtacaattg acg 23

<210> 14
<211> 23
<212> DNA
<213> Artificial Sequence

<220>
<223> oligonucleotide capable of binding specifically to SRSV
RNA

<400> 14
cccaatagtc aacagtttgt ccc 23

<210> 15
<211> 23
<212> DNA
<213> Artificial Sequence

<220>
<223> oligonucleotide capable of binding specifically to SRSV
RNA

<400> 15
aaacccaata gtcaacagtt tgt 23

<210> 16
<211> 20
<212> DNA
<213> Artificial Sequence

<220>
<223> oligonucleotide capable of binding specifically to SRSV
RNA

<400> 16
tcttcttaat cttttgataa                                                    20

<210> 17
<211> 20
<212> DNA
<213> Artificial Sequence

<220>
<223> oligonucleotide capable of binding specifically to SRSV
RNA

<400> 17
taactgttga gagatcagca                                                    20

<210> 18
<211> 20
<212> DNA
<213> Artificial Sequence

<220>
<223> oligonucleotide capable of binding specifically to SRSV
RNA

<400> 18
taaatcattg gaccatcttc                                                    20

<210> 19
<211> 20

<212> DNA
<213> Artificial Sequence

<220>
<223> oligo-4

<400> 19
tgaggcgggc ggggtcaaaa                                                    20

<210> 20
<211> 20
<212> DNA
<213> Artificial Sequence

<220>
<223> oligo-5

<400> 20
tcaccggggg tattatttgg                                                    20

<210> 21
<211> 20
<212> DNA
<213> Artificial Sequence

<220>
<223> oligo-6

<400> 21
cttcaagggg cacttctaca                                                    20

<210> 22
<211> 20
<212> DNA
<213> Artificial Sequence

<220>
<223> oligo-7

<400> 22
cgactgtggg agggactagg                                                    20

<210> 23
<211> 20
<212> DNA
<213> Artificial Sequence

<220>
<223> oligo-10

<400> 23
aggatataat ttgaactcac                                                    20

<210> 24
<211> 20
<212> DNA
<213> Artificial Sequence

<220>
<223> oligo-12

<400> 24
cagcggaaca tagcctgcca                                                    20

<210> 25
<211> 20
<212> DNA
<213> Artificial Sequence

<220>
<223> oligo-13

<400> 25
agattgcgat ctcctgtcca                                                    20

<210> 26

<211> 20
<212> DNA
<213> Artificial Sequence

<220>
<223> oligo-14

<400> 26
agggtcctaa catcagcaat                                                    20

<210> 27
<211> 24
<212> DNA
<213> Artificial Sequence

<220>
<223> 1S

<400> 27
tccttttatg gtatgggtgt cccg                                               24

<210> 28
<211> 24
<212> DNA
<213> Artificial Sequence

<220>
<223> 2S

<400> 28
gggccgcggt gtaaagtggc ttca                                               24

<210> 29
<211> 24
<212> DNA
<213> Artificial Sequence

<220>

<223> 15S

<400> 29
ctaccattct ggaattgaac attt                                              24

<210> 30
<211> 24
<212> DNA
<213> Artificial Sequence

<220>
<223> 8S

<400> 30
attgggtttg acagaattgg tcac                                              24

<210> 31
<211> 51
<212> DNA
<213> Artificial Sequence

<220>
<223> 1F1

<400> 31
aattctaata cgactcacta tagggagaaa aggaaaaatg atgattggaa t               51

<210> 32
<211> 51
<212> DNA
<213> Artificial Sequence

<220>
<223> 1F2

<400> 32
aattctaata cgactcacta tagggagaat aaaaggaaaa atgatgattg g               51

<210> 33
<211> 51
<212> DNA
<213> Artificial Sequence

<220>
<223> 2F1

<400> 33
aattctaata cgactcacta tagggagacg gccctaaagg atgaattagt c     51

<210> 34
<211> 51
<212> DNA
<213> Artificial Sequence

<220>
<223> 2F2

<400> 34
aattctaata cgactcacta tagggagacc gcggccctaa aggatgaatt a     51

<210> 35
<211> 51
<212> DNA
<213> Artificial Sequence

<220>
<223> 15F1

<400> 35
aattctaata cgactcacta tagggagatg gtaggtgtac aattgacggt c     51

<210> 36
<211> 51
<212> DNA
<213> Artificial Sequence

<220>
<223> 15F2

<400> 36
aattctaata cgactcacta tagggagaga atggtaggtg tacaattgac g     51

<210> 37
<211> 51
<212> DNA
<213> Artificial Sequence

<220>
<223> 8F1

<400> 37
aattctaata cgactcacta tagggagacc caatagtcaa cagtttgtcc c     51

<210> 38
<211> 51
<212> DNA
<213> Artificial Sequence

<220>
<223> 8F2

<400> 38
aattctaata cgactcacta tagggagaaa acccaatagt caacagtttg t     51

<210> 39
<211> 24
<212> DNA
<213> Artificial Sequence

<220>
<223> YO-SRSV-S-G

<400> 39
tgcataaatc attggaccat cttc                                   24

## Description

### Field of the Invention

The present invention relates to oligonucleotides for detection of SRSV (Small Round Structured Virus) RNA and to detection methods for SRSV in clinical examinations, public health examinations, food evaluations and food poisoning examinations. SRSV is commonly known as a causative virus of viral food poisoning.

### Prior Art

SRSV belongs to the human Calicivirus group. Human Caliciviruses are classified according to their three genetic types: Genogroup I (GI), Genogroup II (GII) and Genogroup III (GIII). Generally speaking, GI and GII Caliciviruses are generally referred to as SRSV, and GIII Caliciviruses are referred to as human Caliciviruses in the narrow sense.

Approximately 20% of the food poisoning cases reported in Japan are attributed to viral causes. SRSV is detected in over 80% of these viral food poisoning cases. The major source of infection is food, and raw oysters are often implicated. SRSV has also been detected in infant (sporadic) acute enterogastritis, thus suggesting the possibility of propagation from human to human. SRSV detection therefore provides an important contribution to public health and food quality.

To date, SRSV detection has been based on electron microscope observation. Detection by this method, however, requires the virus to be present in an amount of 10⁶/ml or greater, and thus the detection subject was limited to patient feces. Sometimes the virus has not been identifiable even when observed.

In recent years, it has become possible to produce viroid hollow particles for human caliciviruses, and research is advancing on a specific antibody-detecting ELISA employing such particles. However, the detection sensitivity is still on the same level as electron microscopy, and the method is therefore far from highly sensitive.

As mentioned above, since a complex procedure and prolonged periods are required for the conventional method and it is difficult to detect trace amounts of SRSV in samples within short periods, it has been desired to provide a detection method with the high speed and high sensitivity necessary for food evaluation and the like. There has also been a demand for development of an automated examination device which allows more convenient examination.

Methods of amplifying target nucleic acid can be utilized as high sensitivity detection methods. One known method for amplification of specific sequences of genomic RNA such as that of SRSV is the reverse transcription-polymerase chain reaction (RT-PCR). In this method, cDNA for the target RNA is synthesized by a reverse transcription step, and then a cycle of heat denaturation, primer annealing and extension reaction is repeated in the presence of a pair of primers which are complementary and homologous to the ends of specific sequences of the cDNA (the antisense primer may be the one used in the reverse transcription step) and thermostable DNA polymerase, to amplify the specific DNA sequence. However, the RT-PCR method requires a two-stage procedure (reverse transcription and PCR) as well as a repeated procedure of drastic temperature raising and lowering, and these factors have been impediments to its automation.

Other methods known for amplification of specific RNA sequences include the NASBA and 3SR methods which accomplish amplification of specific RNA sequences by the combined effects of reverse transcriptase and RNA polymerase. In these methods, the target RNA is used as a template for synthesis of promoter sequence-containing double-stranded DNA using a promoter sequence-containing primer, reverse transcriptase and Ribonuclease H, this double-stranded DNA is used as a template for synthesis of RNA containing the specific base sequence of the target RNA using RNA polymerase, and then a chain reaction is conducted using this RNA as the template for double-stranded DNA synthesis containing the promoter sequence.

Thus, the NASBA and 3SR methods allow nucleic acid amplification at a constant temperature and are therefore considered suitable for automation. However, since these amplification methods involve relatively low temperature reactions (41°C, for example), the target RNA forms an intramolecular structure which inhibits binding of the primer and is considered to possibly result in a lower reaction efficiency. It has therefore been necessary to subject the target RNA to heat denaturation before the amplification reaction to destroy the intramolecular structure of the target RNA and thus improve the primer binding efficiency.

It is therefore an object of the present invention to provide oligonucleotides that can complementarily bind specifically to regions of target RNA that are free of an intramolecular structure. More particularly, it is an object to provide oligonucleotides that bind to the molecular structure-free regions of genomic RNA of SRSV (especially Chiba virus) at a relatively low and constant temperature (35-50°C, and preferably 41°C), to provide oligonucleotides for detection of SRSV (especially Chiba virus) RNA, i.e. oligonucleotide probes to be used for nucleic acid amplification, and to provide a convenient, rapid and highly sensitive detection method, utilizing the oligonucleotides, for food evaluations and food poisoning examinations.

### Detailed Description of the Invention

The invention of claim 1, which has been accomplished to achieve this object, relates to an oligonucleotide useful for detection of small round structured virus (SRSV) genomic RNA, which is an oligonucleotide capable of specifically binding to SRSV RNA and comprising at least 10 contiguous bases of any of the sequences listed as SEQ. ID. No.1 to No.7.

The invention of claim 2 relates to an SRSV RNA amplification process in which the specific sequence of SRSV RNA present in a sample is used as a template for synthesis of cDNA by RNA-dependent DNA polymerase, the RNA of the RNA/DNA hybrid is decomposed by Ribonuclease H to produce single-stranded DNA, said single-stranded DNA is used as a template for production by DNA-dependent DNA polymerase of double-stranded DNA having a promoter sequence capable of transcribing RNA with said specific sequence or the sequence complementary to said specific sequence, said double-stranded DNA produces an RNA transcription product in the presence of RNA polymerase, and said RNA transcription product is then used as a template for cDNA synthesis by said RNA-dependent DNA polymerase, the RNA amplification process being characterized by employing a first oligonucleotide comprising at least 10 contiguous bases, of any of the sequences listed as SEQ. ID. No.1 to No.7, which are capable of specifically binding to SRSV RNA, and a second oligonucleotide comprising at least 10 contiguous bases, of any of the sequences listed as SEQ. ID. No.8 to No.15, which has a sequence homologous to a portion of the SRSV RNA sequence to be amplified (where either or both the first and second oligonucleotides include an RNA polymerase promoter sequence at the 5' end).

The invention of claim 3 relates to the process of claim 2, wherein said RNA amplification process is carried out in the presence of an oligonucleotide probe capable of specifically binding to the RNA transcription product resulting from the amplification and labeled with an intercalator fluorescent pigment, and changes in the fluorescent properties of the reaction solution are measured (with the proviso that said labeled oligonucleotide has a sequence different from said first oligonucleotide and second oligonucleotide).

The invention of claim 4 relates to the detection method of claim 3, characterized in that said probe is designed so as to complementarily bind with at least a portion of the sequence of the RNA transcription product, and the fluorescent property changes relative to that of a situation where a complex formation is absent.

The invention of claim 5 relates to the detection method of claim 4, characterized in that said probe comprises at least 10 contiguous bases of any of the sequences listed as SEQ. ID. No.16 to No.18 or its complementary sequence.

The present invention relates to oligonucleotides useful for detection of genomic RNA of SRSV, which are oligonucleotides that can bind specifically to SRSV RNA and comprise at least 10 contiguous bases of any of the sequences listed as SEQ. ID. No.1 to No.7. The oligonucleotides can bind specifically to SRSV RNA at relatively low and constant temperature (35-50°C, and preferably 41°C).

The invention provides a nucleic acid amplification process for amplification of SRSV RNA in a sample, and a detection method for RNA transcription products obtained by the nucleic acid amplification process. The amplification process of the invention includes the PCR, NASBA and 3SR methods, but is preferably a constant temperature nucleic acid amplification method such as the NASBA or 3SR method whereby SRSV-specific RNA sequences are amplified by the concerted action of reverse transcriptase and RNA polymerase (a reaction under conditions in which reverse transcriptase and RNA polymerase act in concert).

For example, the NASBA method is an RNA amplification process in which the specific sequence of SRSV RNA present in a sample is used as a template for synthesis of cDNA by RNA-dependent DNA polymerase, the RNA of the RNA/DNA hybrid is decomposed by Ribonuclease H to produce single-stranded DNA, the single-stranded DNA is used as a template for production by DNA-dependent DNA polymerase of double-stranded DNA having a promoter sequence capable of transcribing RNA comprising the specific sequence or the sequence complementary to the specific sequence, the double-stranded DNA produces an RNA transcription product in the presence of RNA polymerase, and the RNA transcription product is then used as a template for cDNA synthesis by the RNA-dependent DNA polymerase, and this process is characterized by employing a first oligonucleotide comprising at least 10 contiguous bases of any of the sequences listed as SEQ. ID. No.1 to No.7 which are capable of specifically binding to SRSV RNA, and a second oligonucleotide comprising at least 10 contiguous bases of any of the sequences listed as SEQ. ID. No.8 to No.15, which has a sequence homologous to a portion of the SRSV RNA sequence to be amplified (where either or both the first and second oligonucleotides include an RNA polymerase promoter sequence at the 5' region).

While there are no particular restrictions on the RNA-dependent DNA polymerase, the DNA-dependent DNA polymerase and the Ribonuclease H, AMV reverse transcriptase which has all of these types of activity is preferred. The RNA polymerase is also not particularly restricted, but T7 phase RNA polymerase and SP6 phage RNA polymerase are preferred.

In this amplification process, there is added an oligonucleotide which is complementary to the region adjacent and overlapping with the 5' end of the specific sequence region (bases 1 to 10) of the SRSV RNA sequence, and the SRSV RNA is cleaved (with Ribonuclease H) at the 5' end region of the specific sequence to prepare the initial template for nucleic acid amplification, thereby allowing amplification of SRSV RNA without the specific sequence at the 5' end. The oligonucleotide used for this cleaving may, for example, be any of those of SEQ. ID. No.1 to No.7 (provided that it differs from the ones used as the first oligonucleotide in the amplification process). The cleaving oligonucleotide is preferably chemically modified (for example, aminated) at the 3' hydroxyl in order to prevent extension reaction from the 3' end.

The RNA amplification product obtained by the aforementioned nucleic acid amplification process may be detected by a known detection method but, preferably, the amplification process is carried out in the presence of an oligonucleotide probe labeled with an intercalator fluorescent pigment, while measuring the changes in the fluorescent properties of the reaction solution. The oligonucleotide probe will typically be one wherein the intercalator fluorescent pigment is bonded to phosphorus in the oligonucleotide by way of a linker. With this type of suitable probe, formation of a double strand with the target nucleic acid (complementary nucleic acid) causes the intercalator portion to intercalate in the double-stranded portion resulting in a change in the fluorescent property, so that no separatory analysis is necessary (Ishiguro, T. et al. (1996), Nucleic Acids Res. 24(24) 4992-4997).

The probe sequence is not particularly restricted so long as it has a sequence complementary to at least a portion of the RNA transcription product, but it is preferably a sequence comprising at least 10 contiguous bases of the sequences listed as SEQ. ID. No.16 to No.18. Also, chemical modification (for example, glycolic acid addition) at the 3' end hydroxyl group of the probe is preferred in order to prevent extension reaction with the probe as a primer.

By carrying out the amplification process in the presence of this type of probe, it is possible to amplify and detect RNA comprising the same sequence as the specific sequence of SRSV RNA in a single tube at a constant temperature and in a single step, thus facilitating its application for automation.

### Brief Description of the Drawings

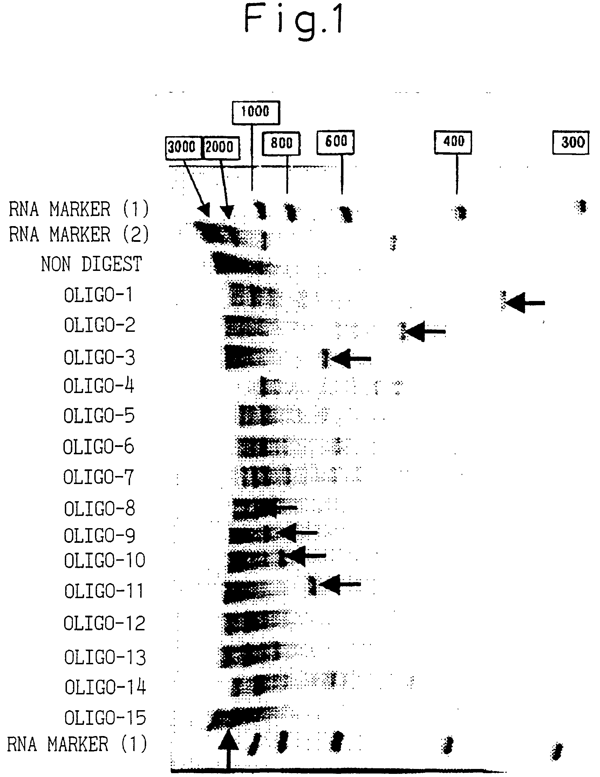

Fig. 1 is a urea modified 6% PAGE electrophoresis diagram for a sample after an SRSV RNA standard binding test at 41°C, using Oligo-1 to Oligo-15 (black and white inverted).

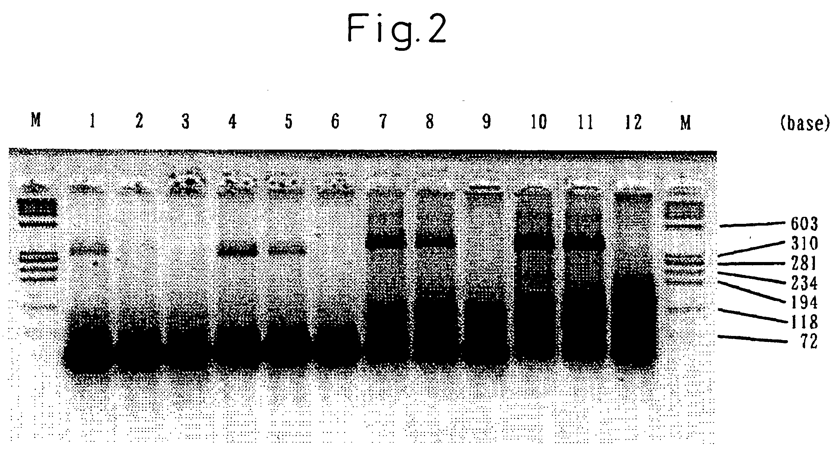

Fig. 2 is an electrophoresis diagram for an RNA amplification reaction in Example 2 using the oligonucleotide combinations (a) to (d) shown in Table 2, with the initial RNA amount of 10⁴ copies/30 µl. Lanes 1 and 2 are the results for combination (a), Lanes 4 and 5 for combination (b), Lanes 7 and 8 for combination (c) and Lanes 10 and 11 for combination (d), while Lanes 3, 6, 9 and 12 are for the Nega (using only the diluent instead of RNA samples). The molecular marker used was φX174/HaeIII digest (Marker 4) (Lane M). Specific bands were confirmed using all combinations.

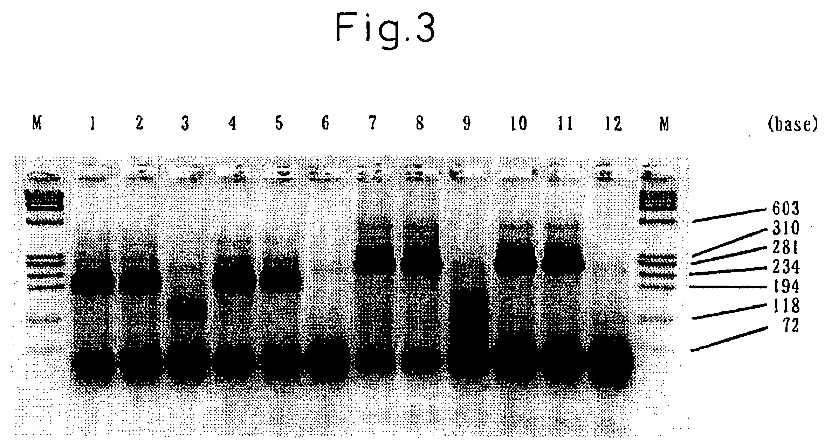

Fig. 3 is an electrophoresis diagram for an RNA amplification reaction in Example 2 using the oligonucleotide combinations (e) to (h) shown in Table 2, with the initial RNA amount of 10⁴ copies/30 µl. Lanes 1 and 2 are the results for combination (e), Lanes 4 and 5 for combination (f), Lanes 7 and 8 for combination (g) and Lanes 10 and 11 for combination (h), while Lanes 3, 6, 9 and 12 are for the Nega (using only the diluent instead of RNA samples). The molecular marker used was φX174/HaeIII digest (Marker 4) (Lane M). Specific bands were confirmed using all combinations.

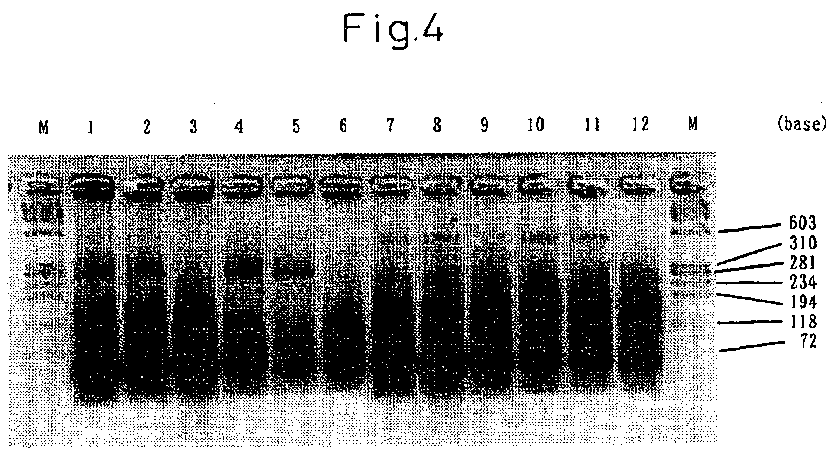

Fig. 4 is an electrophoresis diagram for an RNA amplification reaction in Example 2 using the oligonucleotide combinations (i) to (l) shown in Table 2, with the initial RNA amount of 10⁴ copies/30 µl. Lanes 1 and 2 are the results for combination (i), Lanes 4 and 5 for combination (j), Lanes 7 and 8 for combination (k) and Lanes 10 and 11 for combination (1), while Lanes 3, 6, 9 and 12 are for the Nega (using only the diluent instead of RNA samples). The molecular marker used was φX174/HaeIII digest (Marker 4) (Lane M). Specific bands were confirmed using all combinations.

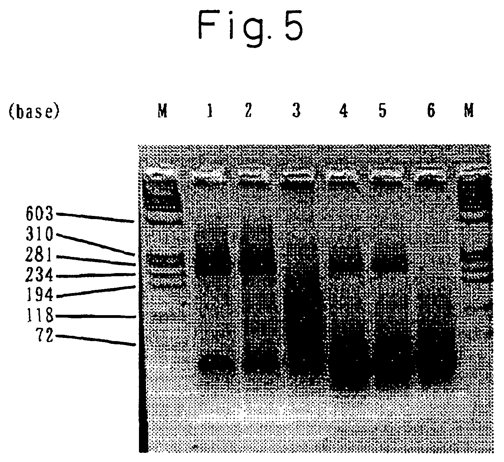

Fig. 5 is an electrophoresis diagram for an RNA amplification reaction in Example 2 using the oligonucleotide combinations (m) and (n) shown in Table 2, with the initial RNA amount of 10⁴ copies/30 µl. Lanes 1 and 2 are the results for combination (m) and Lanes 4 and 5 for combination (n), while Lanes 3 and 6 are for the Nega (using only the diluent instead of RNA samples). The molecular marker used was φX174/HaeIII digest (Marker 4) (Lane M). Specific bands were confirmed using all combinations.

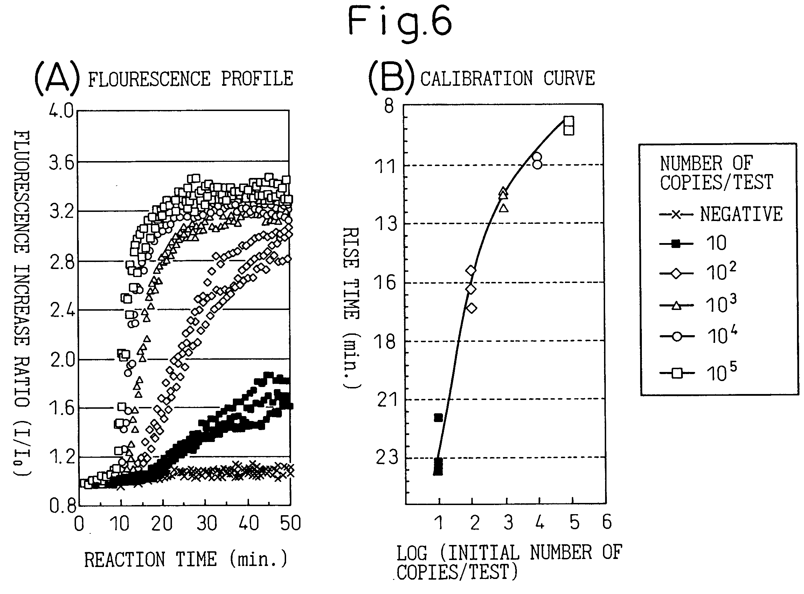

Fig. 6 shows a graph (A) of the fluorescence increase ratio which increases as the reaction time and production of RNA progress, and a calibration curve obtained for the initial RNA amount logarithm and the rise time (B), with an initial RNA amount of between 10¹ copies/30 µl and 10⁵ copies/30 µl in Example 3. The initial amount of 10¹ copies/30 µl of RNA was detectable at approximately 25 minutes of reaction, and a correlation between initial RNA amount and rise time was demonstrated.

### Examples

The present invention will now be explained in further detail by way of examples, with the understanding that the invention is in no way limited by these examples.

### Example 1

An oligonucleotide was selected which binds selectively to SRSV (Chiba virus)at 41°C. Standard RNA of the base sequence of SRSV (Chiba virus) RNA containing bases 1 to 3861 of the structural gene of RNA-dependent RNA polymerase was quantified by ultraviolet absorption at 260 nm, and then an RNA diluent (10 mM Tris-HCl (pH 8.0), 0.1 mM EDTA, 1 mM DTT, 0.5 U/µl RNase Inhibitor) was used for dilution to 0.45 pmol/µl.

A 9.0 µl portion of a reaction solution with the composition shown below was dispensed into 0.5 ml volume PCR tubes (trade name: Gene Amp Thin-Walled Reaction Tubes, product of Perkin-Elmer).

### (Reaction solution composition)

20.0 mM Tris-HCl buffer solution (pH 7.5)
20.0 mM potassium chloride
10.0 mM magnesium chloride
0.1 mM DTT
0.1 mM EDTA
0.9 µM standard RNA
2.0 µM oligonucleotide (oligonucleotide with one of the following sequences)
(Oligo 1): SEQ. ID. No.1
(Oligo 2): SEQ. ID. No.2
(Oligo 3): SEQ. ID. No.3
(Oligo 4): SEQ. ID. No.19
(Oligo 5): SEQ. ID. No.20
(Oligo 6): SEQ. ID. No.21
(Oligo 7): SEQ. ID. No.22
(Oligo 8): SEQ. ID. No.6
(Oligo 9): SEQ. ID. No.7
(Oligo 10): SEQ. ID. No.23
(Oligo 11): SEQ. ID. No.4
(Oligo 12): SEQ. ID. No.24
(Oligo 13): SEQ. ID. No.25
(Oligo 14): SEQ. ID. No.26
(Oligo 15): SEQ. ID. No.5
Distilled water for adjustment of volume

The above reaction solution was incubated at 41°C for 5 minutes, and then 1 µl of 0.1 U/µl RNase H (product of Takara Shuzo Co., Ltd.) was added. (RNase H is an enzyme that cleaves the RNA of DNA/RNA double strands).

The PCR tube was then incubated at 41°C for 15 minutes. In order to identify the cleaved fragments after the reaction, urea-modified polyacrylamide gel (acrylamide concentration: 6%, urea: 7 M) electrophoresis was performed. The dyeing after electrophoresis was accomplished with a commercially available dyeing solution (trade name: SYBR Green II, product of Takara Shuzo Co., Ltd.). When an oligonucleotide binds to the specific site of the target RNA, the RNA of the DNA/RNA double strand is cleaved by RNase H, and a characteristic band is observed.

The results of the electrophoresis are shown in Fig. 1 (black and white inverted image). When the oligonucleotide bound specifically to the standard RNA, the standard RNA decomposed at that region, yielding a decomposition product of a specific chain length. Table 1 shows the positions where the oligonucleotides had specifically bound to the standard RNA and the expected band chain lengths. Specific bands were confirmed for Oligo-1, Oligo-2, Oligo-3, Oligo-8, Oligo-9, Oligo-10, Oligo-11 and Oligo-15. This indicated that these oligonucleotides bind strongly to SRSV RNA under a constant temperature of 41°C. The numbers in Table 1 are provided by designating the initiation base of the pol structural gene as +2, and these positions indicate the 5'-end bases of the (target) region to which each oligo binds. The circles in the table indicate that a specific band was observed, the triangles indicate that a specific band was observed but non-specific bands were also observed, and the "x" symbols indicate that no specific band was observed.

**Table 1**

| Oligo name | Position | Expected band length (base) | Result |
|---|---|---|---|
| Oligo -1 | 358 | 358, 3497 | ○ |
| Oligo -2 | 472 | 472, 3383 | Δ |
| Oligo -3 | 649 | 649, 3406 | ○ |
| Oligo -4 | 1054 | 1054, 2801 | × |
| Oligo -5 | 1720 | 1720, 2135 | x |
| Oligo -6 | 1984 | 1984, 1893 | × |
| Oligo -7 | 2168 | 2168, 1687 | × |
| Oligo -8 | 2560 | 2560, 1294 | ○ |
| Oligo -9 | 2797 | 2797, 1055 | ○ |
| Oligo-10 | 2965 | 2965, 890 | ○ |
| Oligo-11 | 713 | 713, 3142 | ○ |
| Oligo-12 | 1430 | 1430, 2425 | × |
| Oligo-13 | 1455 | 1455, 2400 | x |
| Oligo-14 | 1956 | 1956, 1899 | × |
| Oligo-15 | 2301 | 2301, 1554 | Δ |

### Example 2

Oligonucleotide probes binding specifically to SRSV RNA were used for an RNA amplification reaction.
(1) The same SRSV (Chiba virus) standard RNA used in Example 1 was diluted to 10⁴ copies/5 µl using an RNA diluent (10 mM Tris-HCl (pH 8.0), 0.1 mM EDTA, 0.5 U/µl RNase Inhibitor (product of Takara Shuzo Co., Ltd.), 5 mM DTT). The diluent alone was used for a control test group (Nega).
(2) A 20.8 µl portion of a reaction solution with the composition shown below was dispensed into 0.5 ml volume PCR tubes (trade name: Gene Amp Thin-Walled Reaction Tubes, product of Perkin-Elmer), and 5 µl of the above-mentioned RNA sample was added. Reaction solution composition (Concentrations in 30 µl of final reaction solution)
   60 mM Tris-HCl buffer solution (pH 8.6)
   13 mM magnesium chloride
   90 mM potassium chloride
   39 U RNase Inhibitor
   1 mM DTT
   0.25 mM each of dATP, dCTP, dGTP, dTTP
   3.6 mM ITP
   3.0 mM each of ATP, CTP, GTP, UTP
   0.16 µM of first oligonucleotide
   1.0 µM of second oligonucleotide
   1.0 µM of third oligonucleotide
   13% DMSO
   Distilled water for adjustment of volume
(3) Oligonucleotides with the sequences listed in Table 2 were used as the first, second and third oligonucleotides for RNA amplification reaction. Solutions were prepared so that the combinations of the first, second and third oligonucleotides were the combinations shown in Table 2.
(4) After incubating the reaction solution at 41°C for 5 minutes, 4.2 µl of an enzyme solution with the following composition was added. Enzyme solution composition (Concentrations in 30 µl of final reaction solution)
   1.7% sorbitol
   3 µg bovine serum albumin
   142 U T7RNA polymerase (product of Gibco Co.)
   8 U AMV reverse transcriptase (product of Takara Shuzo Co., Ltd.)
   Distilled water for adjustment of volume
(5) The PCR tube was then incubated at 41°C for 30 minutes. In order to identify the RNA amplified portion after the reaction, agarose gel (agarose concentration: 4%) electrophoresis was performed. The dyeing after electrophoresis was accomplished with a commercially available dyeing solution (trade name: SYBR Green II, product of Takara Shuzo Co., Ltd.). When an oligonucleotide binds to the specific site of the target RNA, the RNA of the portion between the second and third oligonucleotides is amplified, and a characteristic band is observed.

The results of the electrophoresis are shown in Figs. 2 to 5 (black and white inverted images). The chain lengths of the specific bands amplified in this reaction are shown in Table 2. Since specific bands were confirmed for all of the combinations in the RNA amplification reaction using the combinations of oligonucleotides shown in Table 2, it was demonstrated that these oligonucleotides are effective for detection of SRSV.

**Table 2**

| Combination | 1st oligo-nucleotide probe | 2nd oligo-nucleotide probe | 3rd oligo-nucleotide probe | Amplification product chain length (no. of bases) |
|---|---|---|---|---|
| (a) | 1S | 1F1 | 3R | 303 |
| (b) | 1S | 1F2 | 3R | 306 |
| (c) | 1S | 1F1 | 11R | 367 |
| (d) | 1S | 1F2 | 11R | 370 |
| (e) | 2S | 2F1 | 3R | 189 |
| (f) | 2S | 2F2 | 3R | 192 |
| (g) | 2S | 2F1 | 11R | 253 |
| (h) | 2S | 2F2 | 11R | 256 |
| (i) | 15S | 15F1 | 8R | 271 |
| (j) | 15S | 15F2 | 8R | 274 |
| (k) | 15S | 15F1 | 9R | 508 |
| (1) | 15S | 15F2 | 9R | 511 |
| (m) | 8S | 8F1 | 9R | 249 |
| (n) | 8S | 8F2 | 9R | 252 |

Table 2 shows the combinations of first, second and third oligonucleotides used in this example, and the chain lengths of the amplified specific bands resulted from the RNA amplification reaction using those combinations. The 3'-end hydroxyl groups of the first oligonucleotide base sequences were aminated. Of the second oligonucleotide base sequences, the portion of the 1st "A" to the 22nd "A" from the 5'-end is the T7 promoter sequence, and the portion from the 23rd "G" to the 28th "A" is the enhancer sequence.

### First oligonucleotide

1S (SEQ. ID. No.27, base nos. 354-377)

2S (SEQ. ID. No.28, base nos. 468-491)

15S (SEQ. ID. No.29, base nos. 2297-2320)

8S (SEQ. ID. No.30, base nos. 2556-2579)

### Second oligonucleotide

1F1 (SEQ. ID. No.31, base nos. 372-394)

1F2 (SEQ. ID. No.32, base nos. 369-391)

2F1 (SEQ. ID. No.33, base nos. 486-508)

2F2 (SEQ. ID. No.34, base nos. 483-505)

15F1 (SEQ. ID. No.35, base nos. 2315-2337)

15F2 (SEQ. ID. No.36, base nos. 2312-2334)

8F1 (SEQ. ID. No.37, base nos. 2574-2596)

8F2 (SEQ. ID. No.38, base nos. 2571-2593)

### Third oligonucleotide

3R (SEQ. ID. No.3, base nos. 649-668)

11R (SEQ. ID. No.4, base nos. 713-732)

8R (SEQ. ID. No.6, base nos. 2560-2579)

9R (SEQ. ID. No.7, base nos. 2797-2816)

### Example 3

The oligonucleotide combinations according to the invention were used for detection with different initial copy numbers of target SRSV RNA.
(1) The same SRSV (Chiba virus) standard RNA used in Example 1 was diluted to between 10⁵ copies/5 µl and 10¹ copies/5 µl using an RNA diluent (10 mM Tris-HCl (pH 8.0), 0.1 mM EDTA, 0.5 U/µl RNase Inhibitor (product of Takara Shuzo Co., Ltd.), 5 mM DTT). The diluent alone was used for a control test group (Nega).
(2) A 20.8 µl portion of a reaction solution with the composition shown below was dispensed into 0.5 ml volume PCR tubes (trade name: Gene Amp Thin-Walled Reaction Tubes, product of Perkin-Elmer), and 5 µl of the above-mentioned RNA sample was added. Reaction solution composition (Concentrations in 30 µl of final reaction solution)
   60 mM Tris-HCl buffer solution (pH 8.6)
   17 mM magnesium chloride
   90 mM potassium chloride
   39 U RNase Inhibitor
   1 mM DTT
   0.25 mM each of dATP, dCTP, dGTP, dTTP
   3.6 mM ITP
   3.0 mM each of ATP, CTP, GTP, UTP
   0.16 µM of first oligonucleotide (1S, SEQ. ID. No.27, 3'-end hydroxyl group aminated)
   1.0 µM of second oligonucleotide (1F2, SEQ. ID. No.32)
   1.0 µM of third oligonucleotide (11R, SEQ. ID. No.4)
   25 nM of intercalator fluorescent pigment-labeled oligonucleotide (YO-SRSV-S-G, SEQ. ID. No.39, labeled with intercalator fluorescent pigment at phosphorus between 11th "A" and 12th "T" from the 5'-end, and modified with glycol group at 3'-end hydroxyl)
   13% DMSO
      Distilled water for adjustment of volume
(3) After incubating the reaction solution at 41°C for 5 minutes, 4.2 µl of an enzyme solution having the following composition and previously incubated at 41°C for 2 minutes was added. Enzyme solution composition (Concentrations in 30 µl of final reaction solution)
   1.7% sorbitol
   3 µg bovine serum albumin
   142 U T7RNA polymerase (product of Gibco Co.)
   8 U AMV reverse transcriptase (product of Takara Shuzo Co., Ltd.)
      Distilled water for adjustment of volume
(4) The PCR tube was then incubated at 41°C using a fluorescence spectrophotometer equipped with a temperature-regulating function allowing direct measurement, and the reaction solution was periodically measured at an excitation wavelength of 470 nm and a fluorescent wavelength of 510 nm.
Fig. 6(A) shows the ongoing change in the fluorescence increase ratio of the sample (fluorescent intensity value at specified time/fluorescent intensity value of background), where 0 minutes is the time of enzyme addition. Fig. 6(B) shows the relationship between the logarithm of the initial RNA amount and the rise time (time at which the fluorescence increase ratio reaches the value exceeding 3 times standard deviation (3SD) of the average value for the negative:1.2). The initial RNA amount was between 10¹ copies/30 µl and 10⁵ copies/30 µl.
Fig. 6 shows that 10¹ copies were detected at approximately 25 minutes. A fluorescent profile and calibration curve dependent on the initial concentration of the labeled RNA were obtained, indicating that it was possible to quantify the SRSV RNA present in an unknown sample. This demonstrated that rapid, highly sensitive detection of SRSV RNA is possible by this method.

### Effect of the Invention

As explained above, the present invention provides oligonucleotides that bind complementarily to intramolecular structure-free regions of small round structured virus (SRSV) RNA, and a detection method employing them. The invention further provides oligonucleotides for detection of small round structured virus (SRSV) RNA, i.e., oligonucleotide primers and oligonucleotide probes to be used for nucleic acid amplification methods.

Oligonucleotides, for detection of small round structured virus (SRSV) RNA, which bind specifically to SRSV RNA at a relatively low, constant temperature (for example, 41°C), and an SRSV RNA detection method, which is a constant temperature nucleic acid amplification method employing the oligonucleotides, are provided.

## Claims

1. An oligonucleotide, useful for detection of small round structured virus (SRSV) genomic RNA, which is an oligonucleotide capable of specifically binding to SRSV RNA and comprising at least 10 contiguous bases of any of the sequences listed as SEQ. ID. No.1 to No.7.

2. An SRSV RNA amplification process in which the specific sequence of SRSV RNA present in a sample is used as a template for synthesis of cDNA by RNA-dependent DNA polymerase, the RNA of the RNA/DNA hybrid is decomposed by Ribonuclease H to produce single-stranded DNA, said single-stranded DNA is used as a template for production by DNA-dependent DNA polymerase of double-stranded DNA having a promoter sequence capable of transcribing RNA with said specific sequence or the sequence complementary to said specific sequence, said double-stranded DNA produces an RNA transcription product in the presence of RNA polymerase, and said RNA transcription product is then used as a template for cDNA synthesis by said RNA-dependent DNA polymerase, the RNA amplification process being **characterized by** employing a first oligonucleotide comprising at least 10 contiguous bases of any of the sequences listed as SEQ. ID. No.1 to No.7 which are capable of specifically binding to SRSV RNA, and a second oligonucleotide comprising at least 10 contiguous bases of any of the sequences listed as SEQ. ID. No.8 to No.15, which has a sequence homologous to a portion of the SRSV RNA sequence to be amplified, where either or both the first and second oligonucleotides include an RNA polymerase promoter sequence at the 5' end.

3. The process of claim 2, wherein said RNA amplification process is carried out in the presence of an oligonucleotide probe capable of specifically binding to the RNA transcription product resulting from the amplification and labeled with an intercalator fluorescent pigment, and changes in the fluorescent properties of the reaction solution are measured, with the proviso that said labeled oligonucleotide has a different sequence from said first oligonucleotide and second oligonucleotide.

4. The detection method of claim 3, **characterized in that** said probe is designed so as to complementarily bind with at least a portion of the sequence of the RNA transcription product, and the fluorescent property changes relative to that of a situation where a complex formation is absent.

5. The detection method of claim 4, **characterized in that** said probe comprises at least 10 contiguous bases of any of the sequences listed as SEQ. ID. No.16 to No.18 or its complementary sequence.
